Europäisches Patentamt

European Patent Office

Office européen des brevets

(11) Publication number: **0 429 960 B1**

# EUROPEAN PATENT SPECIFICATION

(45) Date of publication of patent specification: **31.05.95**  (51) Int. Cl.⁶: **A61L 2/06**, A61L 2/24

(21) Application number: **90121819.8**

(22) Date of filing: **14.11.90**

(54) **Method and apparatus for steam sterilization of articles.**

(30) Priority: **24.11.89 US 440689**

(43) Date of publication of application:
**05.06.91 Bulletin 91/23**

(45) Publication of the grant of the patent:
**31.05.95 Bulletin 95/22**

(84) Designated Contracting States:
**AT BE CH DE DK ES FR GB GR IT LI LU NL SE**

(56) References cited:
| | |
|---|---|
| **EP-A- 0 134 777** | **EP-A- 0 232 170** |
| **DE-A- 2 710 968** | **DE-A- 3 632 675** |
| **FR-A- 2 335 239** | **FR-A- 2 403 801** |
| **GB-A- 1 296 588** | **GB-A- 2 199 496** |
| **US-A- 3 717 434** | |

**DATABASE WPIL Section Ch, Week 8734, Derwent Publications Ltd., London, GB;Class D09, AN 87-235809**

(73) Proprietor: **SCI-CAN, A DIVISION OF LUX AND ZWINGENBERGER LTD.**
**259 Lakeshore Boulevard East**
**Toronto,**
**Ontario M5T 3T7 (CA)**

(72) Inventor: **Newman, Duncan**
**314 Kenilworth Avenue**
**Toronto,**
**Ontario, M4L 3S8 (CA)**

(74) Representative: **Puschmann, Heinz H.**
**Patentanwalt**
**Puschmann & Borchert**
**Patentanwälte**
**European Patent Attorneys**
**Postfach 10 12 31**
**D-80086 München (DE)**

## Description

This invention relates to a process and apparatus for the steam sterilization of articles. More particularly, this invention relates to a system for the efficient sterilization of dental or surgical instruments or the like.

Flow-through sterilizers of the type used in hospitals or in industrial applications are generally provided with access to a source of saturated steam "on tap" which is used to purge air from a chamber containing the articles to be sterilized and to heat the load until sterilization exposure is complete.

In the usual dental or medical office, it would be physically and economically impractical to have an available steam line, and so sterilization of instruments in these applications has hitherto been carried out using standard autoclaves. An autoclave is typically a relatively large sealed vessel which contains a quantity of boiling water under pressure. Such an instrument is generally provided with a lower exit vent which is initially kept open for a period while water is boiled inside the autoclave to purge the air from the interior. As air is incompatible with and denser than saturated steam, it tends to be pushed out the exit valve ahead of the steam.

Nevertheless, the standard arrangement can lead to appreciable quantities of air being retained in the autoclave chamber, necessitating the simultaneous measurement of both pressure and temperature conditions in the chamber if one is to have confidence in the sterilizing conditions to which the contents are exposed. Imperfect purging of air or an incorrect interpretation of the simultaneous readings of the mutually dependant variables of temperature and pressure could present a biological hazard. On the other hand, unduly high temperatures in the standard autoclave can, with time, damage the contents.

Moreover, in a closed system such as the standard autoclave, or in other known systems which employ a circulating water phase, contaminants from the instruments constantly build up with time, leading to corrosion of the autoclave walls, and possible damage to the contents. A further practical disadvantage of standard autoclaves is their inherent slowness, due to the large heat capacity of their heavy walls and support structures.

It has now been found that the aforementioned disadvantages of standard autoclaves can be overcome by the novel sterilizing system of the invention, which is of a simple construction, adapted to be contained within a relatively small and lightweight benchtop unit. Economy and efficiency of the sterilization process, as well as increased speed of operation over the standard autoclave is achieved by mating a relatively small pressure chamber, preferably in the form of a thin-walled and insulated flat tray, to a controlled electrical boiler which generates steam as needed to produce a sterilizing condition inside the chamber.

In one aspect, the invention is a process for the steam sterilization of articles by the controlled delivery of steam from an electrically heated boiler in communication with a pressure chamber through a steam delivery conduit. The process comprises generating steam by the pulsed injection of a controlled quantity of water into the heated boiler whenever it runs dry. The steam from the boiler is permitted to pass into the chamber while a venting conduit from the chamber is open, to expel the air initially therein. The venting conduit is then closed, so that steam generated in the boiler raises the pressure and the temperature in the chamber. The temperature in the chamber is monitored until the desired sterilization temperature is attained, then the temperature in the chamber is controlled to remain substantially constant at the sterilization temperature for a desired period. This control is achieved by modulating the electric power supply to the boiler heater. At predetermined intervals during pressurization of the chamber and during maintenance of the sterilization temperature within the chamber, the venting conduit from the chamber is opened to cause condensed liquid water from the chamber to be purged therethrough. The venting conduit is then closed and, upon completion of sterilization, the boiler is turned off and the venting conduit opened to exhaust the chamber and return it to atmospheric pressure.

In another aspect, the invention is a system for steam sterilizing articles, comprising a pressure chamber for holding the articles to be sterilized, having an inlet conduit for steam and an outlet conduit; valve means for venting the interior of the chamber; and means for injecting steam into the chamber comprising a controlled electric boiler in communication with the pressure chamber through the steam inlet conduit and a pump operable to inject pulses of water into the boiler to produce steam. The system includes sensor means for monitoring the temperature of steam in the chamber and for detecting dryness of the boiler. The aforementioned process of the invention is carried out by the system of the invention under the control of sequencing means responsive to the various sensor means.

In a final aspect, the invention is a sterilization chamber and associated holder, the sterilization chamber comprising a tray to receive instruments to be sterilized and a lid with a pressure seal between the two. The holder, into which the assembled tray and lid may be slid as a cassette, couples the chamber to an external supply of steam and to a venting conduit, and maintains the tray and lid in pressure tight relationship. In a preferred embodiment, the cassette pressure chamber comprises a shallow bottom tray and a shallow rectangular lid which together form the chamber when the lid is placed over the tray. Perimetrical self-actuating sealing means between the tray and the lid are so configured to define a channel which is closed between the wall of the tray and the wall of the lid in the region of a first end of the chamber, but which partly opens into the interior of the pressure chamber at an opposite end of the chamber. A steam inlet duct at the first end of the chamber communicates with the interior of the channel and feeds steam along the channel towards the opposite end of the chamber where the steam is distributed through the opening portion of the channel, passes over the instruments sterilizing them and is then vented through an outlet duct in communication with the interior of the chamber positioned nearby the inlet duct.

Other objects and advantages of the invention will be evident from the following description referring to the accompanying drawings illustrating the process of the invention, a sterilization system according to the invention, and a pressure chamber according to the invention, in which corresponding components are identified with the same reference numerals. In the drawings:

Figure 1 illustrates schematically and in cross-section the arrangement of components in a sterilizing system according to the invention, excluding the sequencing means and conventional interfacial relays controlling operation of the system;

Figure 2 is a detailed view of the location inside the boiler of Figure 1 where the tip of a temperature sensor enters;

Figure 3 is an idealized graphical representation of various operational parameters in the process of the invention as a function of time over the course of a sterilization cycle;

Figure 4 is a schematic representation of the system of the invention, including microprocessor sequencing (controlling) means and the connections to operating components of the system, in which a heavy line connection represents a flow of water, steam or air and the light lines represent electrical connections;

Figure 5 is a flow-chart for electronic microprocessor control apparatus to control the operation of the system of Figure 1, illustrating the steps of the process of the invention;

Figure 6 is a further flow-chart for electronic microprocessor control apparatus, including the additional function of a deviation check, illustrating a further embodiment of the process of the invention;

Figure 7 is an exploded schematic view of a cassette sterilization chamber and holder according to the invention;

Figure 8 is a sectional view from one side of the assembled cassette sterilization chamber of Figure 7;

Figure 9 is a sectional view from one side of the cassette sterilization chamber of Figure 8, shown partially inserted into the associated holder;

Figure 10 is a sectional view from one side of the cassette sterilization chamber of Figure 8, shown fully inserted into the associated holder;

Figure 11 is a perspective view of the bottom tray of the cassette sterilization chamber holding sterilized instruments following completion of a sterilization process;

Figure 12 is a sectional plan view of a preferred embodiment of cassette sterilization chamber according to the invention;

Figure 13 is a sectional view, in perspective, taken along the line 13-13 in Figure 12;

Figure 14 is a detailed partial view in perspective of the arrangement of conduits and seals in the pressure chamber of Figure 12, viewed generally along direction X;

Figure 15 is a detailed plan sectional view of the inlet and outlet area in the pressure chamber of Figure 12;

Figure 16 is a three-dimensional detailed sectional view generally taken along the line 16-16 in Figure 12;

Figure 17 illustrates the installation of the pressure chamber of Figure 12 into insulated holding means for connection to steam inlet and venting conduits in the system of the invention; and

Figure 18 is a further view in vertical section of the pressure chamber and holding means of Figure 17.

Efficiency, rapidity and economy in the sterilization of dental and surgical instruments and the like is achieved according to the apparatus and process of the invention by providing a one-way flow system in which a sterilization chamber is fed with steam as required from a boiler operating as a "flash" boiler.

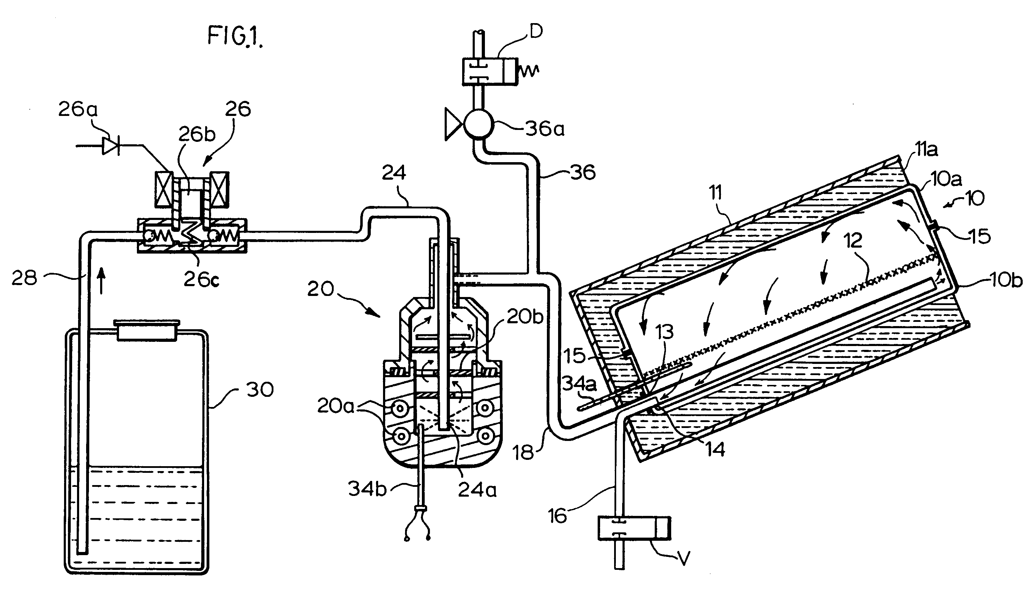

Figure 1 illustrates semi-schematically in vertical cross-section a preferred embodiment of a system according to the invention for the steam injection sterilization of articles in a pressure chamber 10.

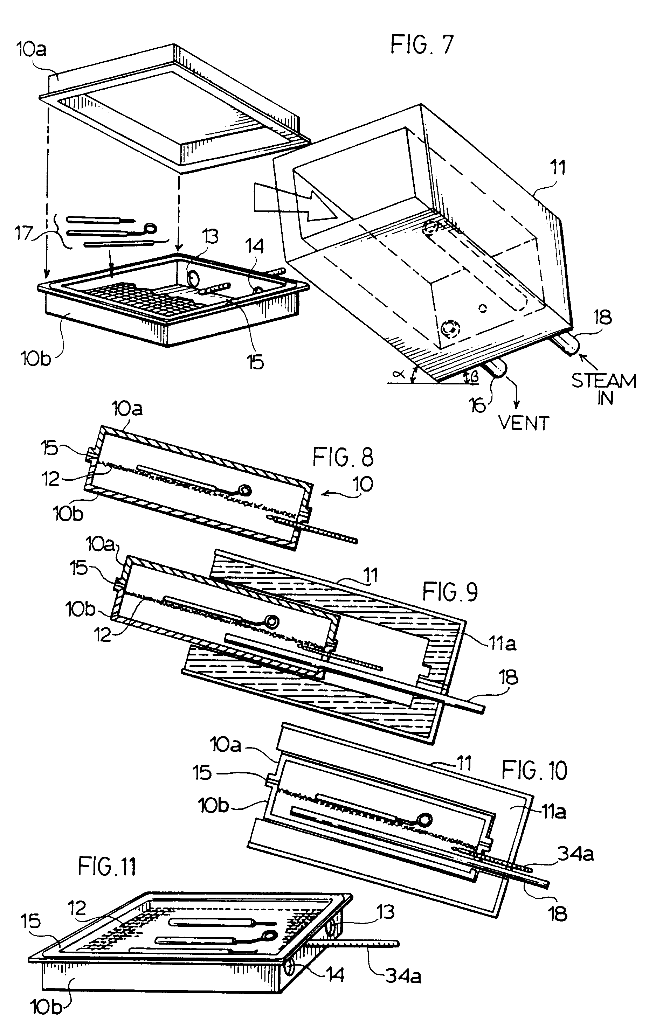

It is emphasized that the utility of the steam sterilization process of the invention and of the system of the invention are not restricted to one particular form of pressure chamber or autoclave for holding the articles to be sterilized. According to one aspect of the invention, however, described in further detail below in connection with Figure 7 to 11 there is provided and shown in use in the system of Figure 1, a novel sterilization chamber in the form of a "cassette" that can be inserted into a rigid holder 11 with an insulating lining 11a, designed to couple the inserted chamber to a steam inlet 18 and a vent conduit 16. The cassette comprises a tray 10b to receive the instruments and a lid 10a with compressible pressure sealing means 15 between the two. The lid 10a can be simply lifted off the tray 10b for loading and unloading, but the holder 11 is designed to maintain the lid and tray in sealing relationship when the cassette is placed in the holder.

Because the system of the invention operates by first purging air from the chamber then sealing the chamber and injecting steam in a controlled manner to bring the chamber interior up to a sterilization temperature, steam should enter the chamber at an end remote from the outlet from the chamber to assist in the initial purging of air therefrom. The outlet port, indicated at 14 in Figure 1, should be disposed at the lowest point in the chamber 10 to permit purging of the liquid water that forms when sterilizing steam condenses in heating the articles in chamber 10, which are preferably laid out prior to sterilization on a perforated grid or rack 12 inside the chamber. In the case of a cassette pressure chamber 10 as illustrated in Figure 1, this can simply be achieved by having the chamber mounted in use at an angle such that condensate runs under gravity toward the outlet, but it will be appreciated that any of a number of simple arrangements and construction of pressure chamber will achieve this, any of which must, of course, provide for access of instruments into the chamber prior to commencement of steam injection, and means for sealing the chamber during use.

In the illustration of Figure 1, chamber 10 is provided at the bottom thereof with an inlet port 13 and an outlet port 14. The outlet port sealingly receives an outlet conduit 16 including a first valve in (for "venting") which may be switched between an open position in which steam, air or condensed water may pass from inside chamber 10 through conduit 16 and into a waste water container. Steam injection conduit 18 is sealingly received in inlet port 13.

The sequence of steps carried out in a sterilization process according to the invention requires the ability to monitor the temperature inside the chamber 10 when it is being pressurized by steam or is being held at the desired steam sterilization condition. As seen in Figure 1, a thermocouple or thermistor 34a extends into chamber 10 in the vicinity of outlet port 14 when the cassette chamber is connected to its inlet and outlet conduits. The signal from thermocouple sensor 34a provides the requisite measure of temperature.

As seen in Figure 1, boiler 20 includes a water injection conduit 24 leading from a dosing pump 26, which may be actuated to draw distilled water through feed line 28 from a container 30 of distilled water and inject a pulse of water of a preset size into boiler 20. As a dosing pump, there may be employed any low-volume high-pressure pump operable to inject a pulse of water when actuated. A preferred component for this purpose is a solenoid plunger pump, whose power input includes a rectifying diode 26a to provide unidirectional pulsing of the pump plunger 26b against a biasing spring 26c.

Boiler 20 is heated by electrical heating elements 20a supplied with electrical power from a switchable power source (not shown). In a typical installation for use in the sterilizing of dental or surgical instruments, boiler 20 will have an interior capacity of around 50 ml., with heating elements 20a providing around 1 kW of power.

It is important that the boiler capacity be sufficiently small relative to the volume of water pulsed into it by each injection from dosing pump 26 to ensure early and complete purging of air from the boiler interior. This is achieved if the volume of water dosed into the boiler is not much less than about 20 percent of the boiler volume. Ideally, the interior volume of the boiler should be no greater than necessary to permit free boiling of the water pulses pumped into it.

Inside the boiler for rapid conversion to steam. Distilled or demineralized water should be used to avoid the build-up of mineral deposits around the below-described dryness sensing means in the boiler. The interior space of the boiler may advantageously be provided with a plurality of stainless steel baffles 20b, to prevent splashover of boiling water into steam injection conduit 18 and to minimize the formation of aerosol droplets of water, which would not transfer heat of vaporization to articles in pressure chamber 10. "High quality" steam having a low aerosol content is thereby fed into the steam injection conduit 18.

The opening and closing of valve V in may be controlled by using as valve V a solenoid two-way valve with conventional electrical switching means (not shown). Pump 26 may likewise be actuated to inject a pulse of water by means of a conventional solenoid and switch arrangement.

In addition to monitoring the temperature inside the chamber 10, it is necessary for the process of the invention to be able to detect when boiler 20 has run dry, so that a fresh pulse of water may be injected using dosing pump 26. The means for detecting dryness of the boiler in the embodiment illustrated in Figure 1 comprises a second temperature sensor 34b such as a thermocouple or thermistor, fixed within the casing of the boiler at a position intermediate heating elements 20a and the interior cavity of boiler 20. Evaporation of water in the boiler to dryness is then detected by an abrupt increase in the sensed temperature as a function of time.

For that embodiment of the process further including a "deviation check" as described below, it is also advantageous for sensor 34b to project a small distance into the interior cavity of boiler 20, so that sensor 34b is in contact with the casing metal, but also exposed to the steam generated within the boiler, as indicated in Figure 1. By this arrangement, the sensor is operable not only to detect abrupt heating of the casing when the boiler runs dry, but also to provide a measure of the equilibrium steam temperature (boiling point) inside the boiler which may be compared with the steam temperature in the chamber 10.

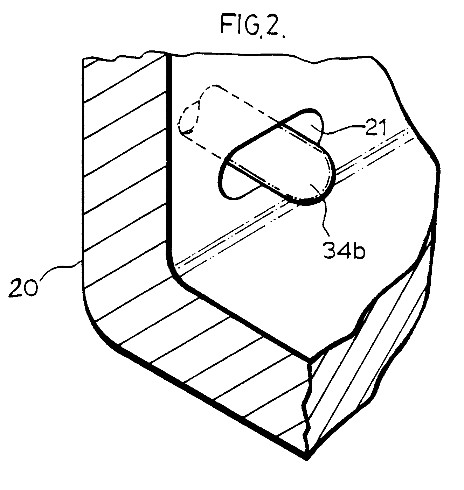

One arrangement for permitting thermocouple 34b to contact the boiler casing while exposed to steam therein is illustrated in Figure 2. The tip of the sensor is surrounded by a shallow out-of-round recess 21 formed in the casing wall, so that it contacts the top and bottom inner surfaces of recess 21, which at the same time permits the access of steam around much of the sensor tip's surface.

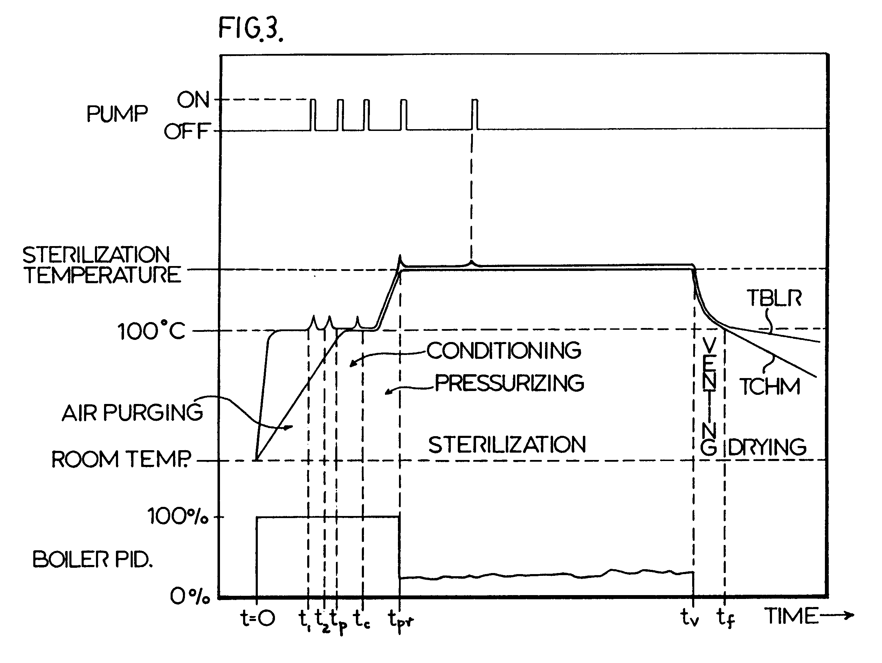

Figure 3 presents four idealized graphical representations of the concurrent variation with time of parameters of operation. From top to bottom, these are: (1) the pulsed injection of water into the boiler, (2) the temperature reading, TBLR from a boiler temperature sensor 34b installed as in Figure 2, (3) the temperature reading TCHM from the chamber thermocouple 34a, and (4) the control of power delivered to the boiler heating elements 20a.

After a time $t_1$ has elapsed following injection of a pulse of water into the boiler, evaporation of the water to dryness is signalled by a sharp increase in the slope of the boiler temperature TBLR. Injection of a short-duration pulse of water at that time drops the value of TBLR until some later time $t_2$ when the injected pulse has evaporated and the dryness signal calls on pump 26 to inject a further pulse of distilled water, and so on whenever dryness of the boiler is detected.

The process of steam sterilization of articles according to the invention will now be described in connection with the embodiment of the system of the invention illustrated in Figure 1 and the time graphs of Figure 3:

At the outset of the sterilization cycle, indicated as $t = 0$ on the graphs of Figure 3, the boiler power is turned on and a pulse of water is injected, generating steam. A flow of steam is generated in boiler 20 by the pulsed injection by pump 26 of a controlled quantity of water into the hot boiler, whenever dryness of the boiler is detected.

In the initial purging phase, from $t = 0$ to $t = t_p$, the steam generated in boiler 20 passes through steam injection conduit 18 into chamber 10 to expel the air initially therein through outlet conduit 16, by way of opened valve V. The importance of effective air removal in steam sterilization processes is well known. A novel pressure chamber according to the invention, which is particularly efficient in this respect, is described in detail below in connection with Figures 7 to 18.

In the purging phase of the sterilization cycle, the air initially present in the chamber is expelled and TCHM rises from room temperature to around 100°C, the equilibrium temperature of saturated steam at atmospheric pressure.

As steam is allowed to pass through the chamber during the purging phase, the temperature of the effluent stream vented from the pressure chamber is monitored using sensor 34a. Once that temperature has risen to about 100°C, indicating substantial purging of air from the chamber, steam is allowed to pass through for a selected conditioning period, from $t = t_p$ to $t = t_c$ in Figure 3, to ensure the elimination of air from the system, and then valve V is closed, so that steam generated in boiler 20 commences to pressurize the chamber. During the pressurization phase, from $t = t_c$ to $t = t_{pr}$ in Figure 3, as in the previous purging phase, boiler 20 remains turned on at its full power and steam continues to be generated from the pulses of water injected into the boiler by pump 26.

During this pressurization phase of the cycle, the interior of the boiler and the pressure chamber form a closed system and go up in temperature together as the equilibrium temperature for saturated steam rises with increasing pressure. Over this period, the difference TBLR - TCHM is a more sensitive measure of the onset of dryness in the chamber than is TBLR alone.

The temperature of the chamber is monitored until a desired sterilization temperature is attained. The selected temperature of sterilization may be as low as about 124°C, when the articles being sterilized are instruments including rubber or thermoplastic components susceptible to damage at higher temperatures. When the articles are simple stainless steel dental instruments, a sterilization temperature of up to around 145°C may be used, allowing for correspondingly reduced sterilization exposure times. The point of attainment of the selected sterilization temperature is shown to have occurred at time $t = t_{pr}$ in Figure 3. From that point until the completion of sterilization exposure of articles in the chamber (at time $t = t_v$) in the chamber is controlled to remain substantially constant at the sterilization temperature.

This sterilization temperature control may be effected by switching the electric power supplied to the boiler cyclically off and on. Computer sequencing means controlling the operation of the system may readily be programmed to monitor a moving average of TBLR and to adjust the duty cycle of power delivered to the boiler heaters as necessary to minimize variations in TCHM. This is referred to in Figure 3 as the PID (proportional integral derivative) control of the boiler duty cycle.

Throughout the pressurization and sterilization phases, pump 26 continues to deliver controlled quantities of water in response to sensed dryness of the boiler. The amount of steam produced increases with the mass to be heated to equilibrium with the saturated steam. Production of steam "on demand" in this manner is far less wasteful of both water and energy than known sterilizing systems. For a 2 litre capacity sterilization chamber constructed as described below, used in the above-described sterilization system employing a 50 ml. boiler, only about 70 mls of distilled water is used up in sterilizing a typical tray of surgical or dental instruments.

Upon completion of the desired sterilization period at time $t = t_v$ vent valve V is opened to exhaust the chamber and return it to atmospheric pressure, and the boiler is turned off. Following venting of the chamber and its return to atmospheric pressure ($t = t_v$), the sterilized instruments may be cooled to a usable temperature simply by removing and opening chamber 10. Alternatively, the system in the embodiment of Figure 1 is provided with a source of clean, dry compressed air connected to an air conduit 36 and regulator 36a branching from steam injection conduit 18. At the conclusion of the sterilization cycle, compressed air may be admitted to line 36 and thence into the chamber via conduit 22, by actuating valve D in the compressed air line. Compressed air (which may first be pre-warmed and/or passed through suitable microbial filters) is then passed over the instruments to hasten cooling and drying (time $t \geq t_f$ in Figure 3).

Alternative arrangements for cooling down the articles in the chamber at the conclusion of sterilization will be readily apparent to those of ordinary skill in the art. Thus, for example, if chamber 10 were provided with check valve means or with self-actuating sealing means operable to admit a flow of atmospheric air into the chamber only when the pressure inside the chamber is below ambient pressure, then a vacuum system could be used to draw out excess condensate from the outlet conduit and draw cooling air into the chamber. The vacuum could be produced by an appropriate vacuum pump. Alternatively, boiler dosing pump 26 of Figure 1 could itself be shunted to power a jet pump (eductor) to draw a vacuum by aspiration. Such an eductor pump might alternatively be powered by a jet of steam from boiler 20.

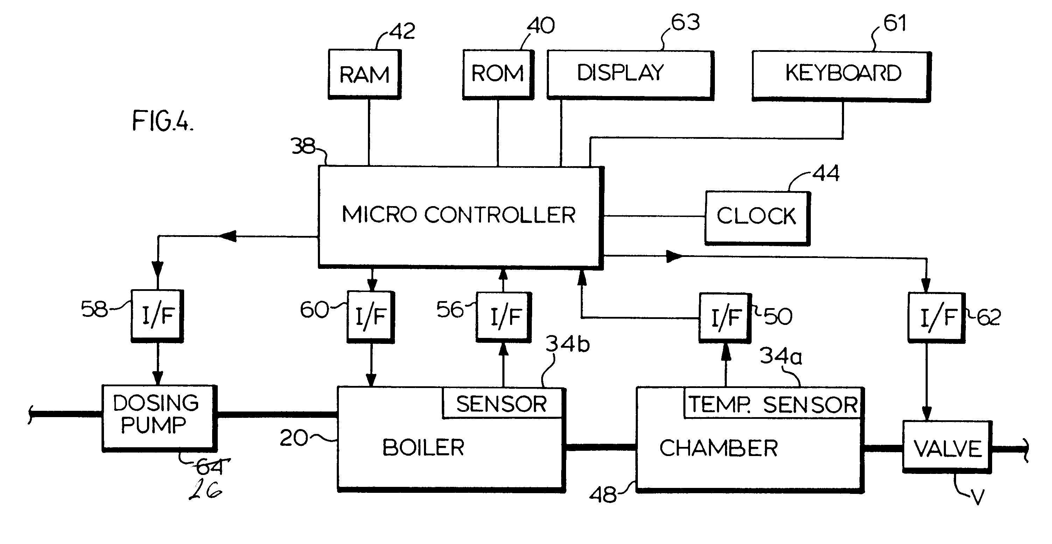

Figure 4 illustrates electronic processor apparatus for controlling the operation of the system of Figure 1 according to the process of the invention.

Microcontroller 38 includes a program memory 40, working memory 42 and a timer clock 44. The microcontroller is responsive to the temperature sensor 34a of the chamber 10 through interface 50 and to the dryness sensor 34b of boiler 20 through interface 56. Output signals from the microcontroller are directed to interfaces 58, 60 and 62 to control the operation of dosing pump 26, heating elements 20a of boiler 20, and valve V, respectively, to carry out the stages in a cycle of steam sterilization. Selected program parameters, such as the operating sterilization temperature, are entered by keyboard terminal 61. Information as to the status of the running sterilization program, such as the time remaining on a clock cycle, is displayed on terminal 63.

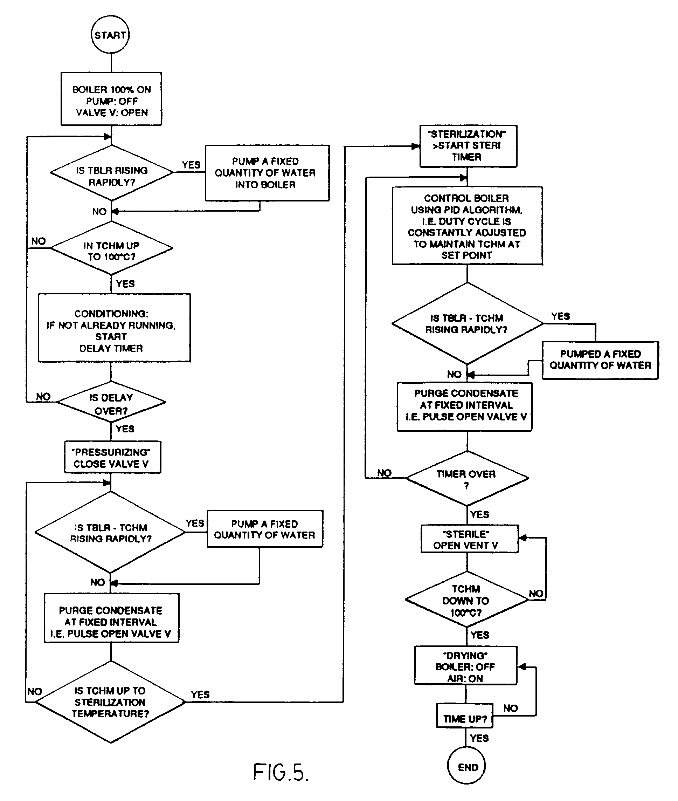

A flow-chart for the operation of the system of Figure 1 under the control of such electronic sequencing means is given in Figure 5. The operation identified as "Purge Condensate at Fixed Interval" relates to the step of opening valve V when the chamber is being pressurized, or during the sterilization period, at suitable intervals to vent condensate from the chamber and to close valve V when the release of gaseous steam from the chamber immediately upon expulsion of the condensate is sensed by a drop in the chamber temperature. By this expedient, the build-up of condensed water is precluded.

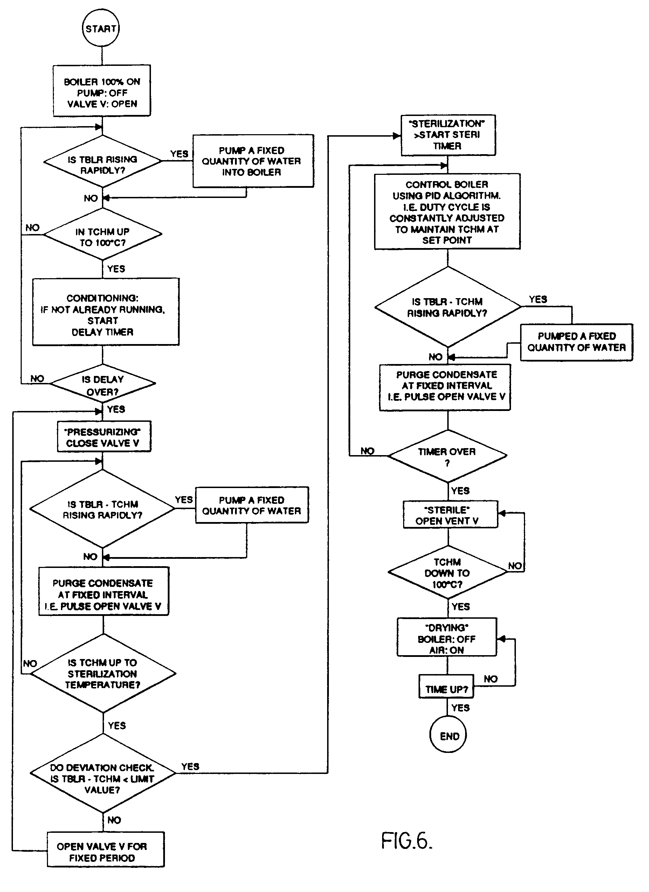

Figure 6 is a similar flow-chart to that of Figure 5, but represents a control process which includes a further sequence loop involving the decision "Do Deviation Check". This is an additional safety feature which addresses the possibility that not all of the air has been purged from the chamber during the conditioning phase, as follows:

The small interior volume of the boiler and the continued boiling of water therein ensures that all air has been purged from the boiler itself shortly after the commencement of boiling, so that TBLR thereafter measures the steam table value of steam in the boiler, i.e. the boiling temperature of water in the absence of air at the given pressure. If substantially all of the air in the chamber has been purged during the purging and conditioning phases of the process, then TCHM will not depart markedly from TBLR, as illustrated in Figure 3. If, however, any undesired air should remain in the chamber during sterilization, this will be reflected in a difference between the equilibrium temperature within the boiler and within the chamber, TBLR > TCHM, since the total pressure in the chamber is the sum of the partial pressures of air and saturated steam.

Accordingly, by the deviation check of the modified process diagrammed in Figure 6, a comparison is made between TCHM and TBLR, taking a running average of both temperatures and ignoring the short-duration "peaks" in TBLR when the boiler runs dry. A value of this difference greater than a predetermined value indicates that there is excess air as well as saturated steam in the system. Vent valve V is then opened for a short fixed period of time to purge the unwanted air from the chamber, and sterilization is recommenced. The deviation check thus permits the control system to monitor and, if necessary, automatically ensure sterilization conditions in the chamber, without having to measure pressure directly anywhere in the system, by contrast with the standard autoclave chambers referred in the earlier discussion of prior art.

As discussed briefly in connection with Figure 1 and the associated description of the system of the invention, there is provided according to the invention a novel sterilization chamber in the form of a cassette that can be plugged into a holder for coupling the chamber to the steam inlet and venting conduits of the system. In its broadest aspects the components and function of the novel cassette arrangement are illustrated schematically in Figure 7 to 11.

As best seen in the exploded view of Figure 7, and the assembled cassette in Figure 8, the cassette sterilizing chamber 10 includes a bottom tray 10b preferably including a grid or perforated rack 12 resting in and spaced above the floor of the bottom of tray 10b for receiving dental instruments 17 or the like and enhancing their exposure to sterilizing steam in the chamber. The cassette 10 further includes a lid 10a and a compressible sealing member 15 between lid 10a and 10b. The lid, bottom tray and rack should be constructed of a material exhibiting strength and resistance to attack by steam, such as stainless steel or anodized aluminum.

As best seen in Figure 7 and in Figures 9 and 10, the latter two showing respectively partial and complete insertion of cassette 10 within holder 11, chamber 10 is provided with lower steam inlet and outlet ports 13 and 14, respectively. Steam injection conduit 18 and outlet conduit 16 plug sealingly into inlet and outlet ports 13 and 14, respectively when cassette 10 is fully inserted into holder 11, by O-rings or other conventional sealing members (not shown).

Rigid holder 11, which may be made of stainless or structural steel, has an inner insulating covering 11a to minimize loss of heat from cassette sterilization chamber 10 during operation. The small clearance between the top of lid 10a and the bottom of tray 10b and, respectively, the upper and lower inner surfaces of the insulating lining of holder 11 is chosen so that compressible sealing member 15 maintains its seal within holder 11 when chamber 10 is pressurized, but permits cassette chamber 10 to be withdrawn freely after sterilization when the chamber is vented or evacuated. Lid 10a is then removed, leaving the sterilized instruments in tray 10b for easy transportation, as illustrated in Figure 11.

In the cassette illustrated in Figures 7 to 11, there is also shown the temperature sensor 34a required for monitoring the chamber temperature during sterilization when cassette 10 is used in the sterilization system of Figure 1, and an aperture 35 in holder 11 for receiving the temperature sensor when the cassette 10 is inserted therein. When cassette 10 is used in steam sterilization, as for example in the controlled injection system of Figure 1, sterilizing steam enters through conduit 18 into the rear of chamber 10. The flow of air and of condensed water toward outlet port 14 is directed by gravity through the expedient of mounting holder 11 at orthogonal angles $\alpha$ and $\beta$ out of the horizontal plane to make the corner of chamber 10 at outlet port 14 the lowest point.

A cassette sterilization chamber and holder as described above presents a number of advantages over the conventional autoclave typically used in the sterilization of instruments in dental or medical offices. Being in the form of a light weight, thin-walled tray inserted into an insulating jacket, the cassette chamber heats up quickly and uses less power in a sterilization cycle than a closed system autoclave. Pressurization of the cassette chamber can be carried out only when the cassette is fully inserted into the fixed rigid holder, eliminating the need for elaborate safety locking mechanisms as are required in autoclaves provided with doors. By its construction and manner of use, the cassette and holder arrangement of the present invention provide for sterilization of the very tray in which the instruments are cooled down for immediate use. No transfer from the interior of a sterilization chamber to an auxiliary carrying tray or other post-sterilization handling, of the kind necessary with conventional autoclaves, is involved.

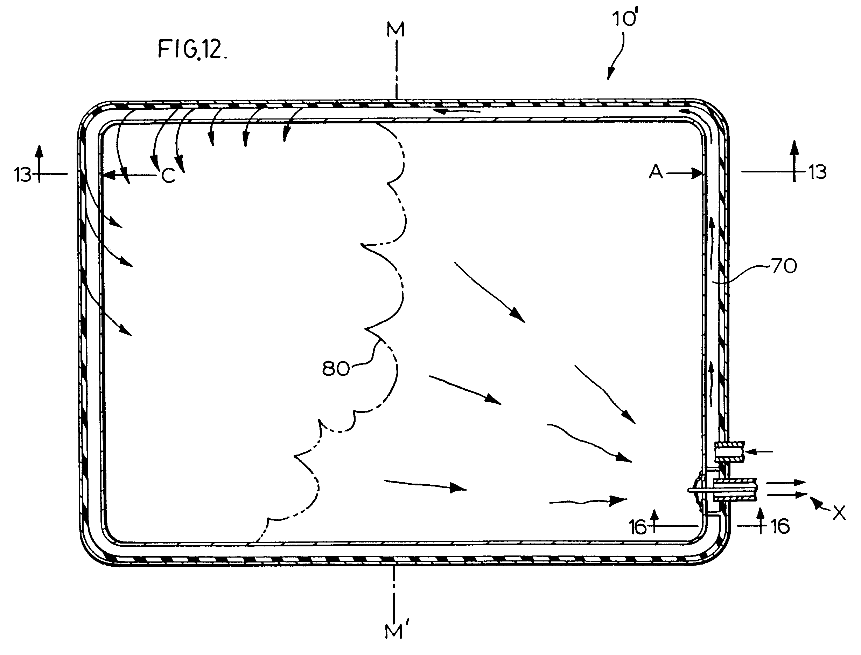

In order to obtain effective purging of air from the chamber, it is desirable that the chamber be designed so as to minimize turbulent mixing of air initially present in the chamber with the sterilizing steam introduced into the chamber. A preferred embodiment of cassette sterilization chamber which is conducive to much better expulsion of air than a conventionally shaped autoclave and is of particular use in obtaining effective purging of air when used in conjunction with the system of Figure 1 is illustrated in Figures 12 to 18. Structural components of this preferred embodiment of cassette sterilizer which are analogous to those of the cassette sterilizer of Figure 7 to 11 are given the same reference numeral, but primed. Thus lid "10a" in Figure 7 to 11 corresponds to lid "10′a" in Figures 12 to 18.

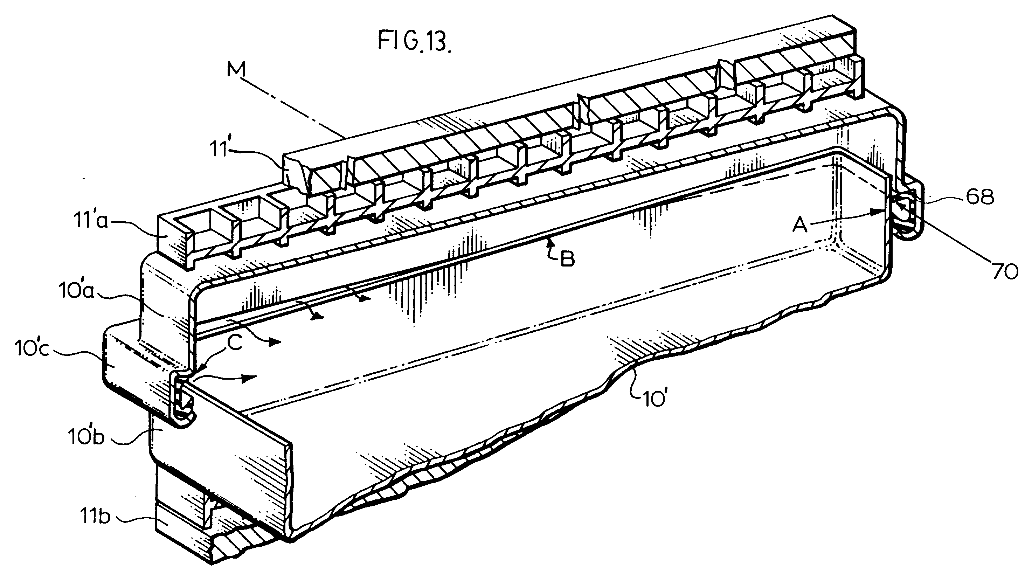

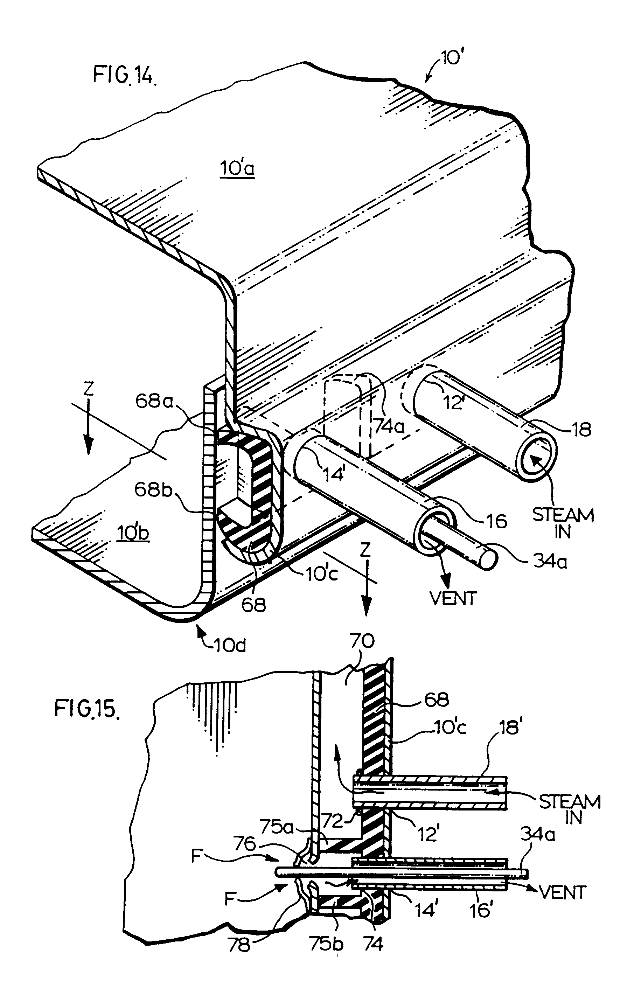

As best seen in Figures 13 and 14, the chamber 10′ comprises a lid 10′a and a bottom tray 10′b which holds dental or surgical instruments or other articles to be sterilized. Preferably, the instruments may be placed on or in a permeable holder (not shown) as in the cassette of Figure 7 to 11. Again, the lid, bottom tray and rack should be constructed of a material exhibiting strength and resistance to attack by steam, such as stainless steel or anodized aluminum.

Fitted within an outwardly stepped perimetral channel 10′c at the base of lid 10′a is a flexible, unitary seal 68 having a generally J-shaped cross-section. As best seen in Figure 14, when lid 10′a is installed over bottom tray 10′b in operation, the extremities of the J-seal contour abut against the outer wall of tray 10′b, as at points 68a and 68b, to form an interior channel 70 along the length of seal 68 closed off from the outside of chamber 10′. The shape and flexibility of seal 68 are such that pressurization of channel 70 by steam actuates the sealing action by flexing the lower wall of the seal more firmly against the tray. The principle of such self-actuating or self-energizing seals is well known and employed, for example, in pressure cookers and the lip seals used in hydraulic cylinders.

Lid 10′a is slightly wider and longer than bottom tray 10′b, but is prevented from slipping below its operating position by matching exterior stops on the lid and tray (not shown). Lid 10′a includes at one lower corner thereof ports 12′ and 14′ to receive and hold a steam injection conduit 18′ and a venting conduit 16′, respectively. In operation, a thermocouple 34a is inserted in venting conduit 16′.

In the preferred embodiment of cassette chamber of the invention, as best seen in Figure 15, the self-actuating seal includes an inlet aperture 72 that is aligned with chamber wall inlet port 12' when lid 10′a is in place over tray 10′b, to receive the steam inlet conduit 18′. Steam injected into the chamber proceeds along channel 70 in the direction of the successive positions A, B and C indicated in Figures 12 and 13.

The seal 68 is formed with an outlet aperture 74 that aligns with lid wall outlet port 14′ to receive the outlet (venting) conduit 16′. However, disposed to the sides of the outlet aperture 74 are a pair of integral partitions 75a and 75b, which close aperture 74 off from seal channel 70.

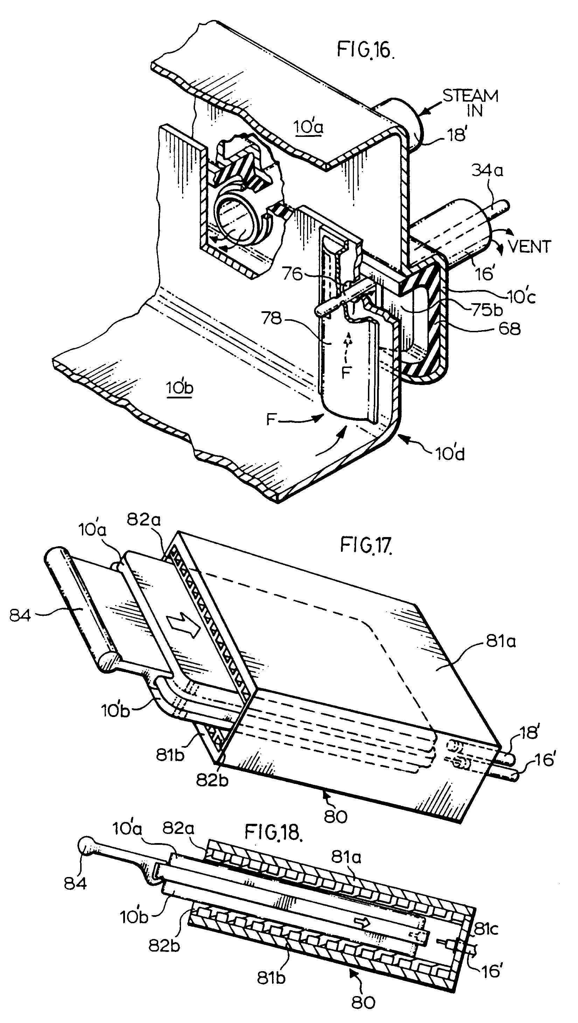

As best seen in Figures 15 and 16, the interior of chamber 10′ communicates with the vent conduit 16′ through a tray wall aperture 76 aligned with lid wall outlet port 14′ and seal outlet aperture 74. The effluent stream of gases and/or condensate purged from the interior of chamber 10′ exits the chamber from the region of the bottommost corner 10′d, through a vertical channel member 78 open at the bottom near the floor of tray 10′b. The outflow of steam and condensate through channel member 78 is indicated by arrows F in Figures 15 and 16. Temperature sensor 34a may extend a short distance into the interior of chamber 10′ through a peripherally sealed aperture in channel member 78, to hold it in place within the effluent stream.

As best seen in Figures 12 and 13, the plane of the lid channel 10′c that holds seal 78 is tilted downwardly with respect to the upper edge of the bottom tray 10b about the transverse axis MM′, so that when lid 10′a is set in place on tray 10′b, channel 70 is entirely occluded by the vertical side walls of tray 10b to that side of axis MM′ disposed toward the steam inlet and outlet conduits, but communicates with the interior of the chamber to an increasing degree proceeding to the other side of axis MM′.

Thus, at position A along the channel, the seal 68 is entirely below the upper edge of tray 10′b. At position B, the upper contact of seal 68 with the tray just meets the upper edge of the tray, and by position C is displaced above that upper edge, so that steam initially introduced within channel 70 vents into the interior of the chamber.

The result of this arrangement of seal and chamber components is that steam injected into the seal channel 70 at one corner of the chamber 10 enters the tray along the opposite corner of the chamber and proceeds across the chamber toward the vent. A high-velocity flow of steam from the boiler is thereby converted to a low-velocity piston-like front of steam 80, which efficiently and without turbulence pushes chamber atmosphere out ahead of it.

Figures 17 and 18 illustrate the holding means for use in association with the pressure chamber of Figures 12-18. Indicated generally at 80, the holder comprises a pair of retaining plates 81a and 81b having respective insulated coverings 82a and 82b with textured surfaces, such as small rectangular bosses. This arrangement minimizes loss of heat from chamber 10′ during operation. Too, the spacing between chamber 10′ having lid 10′a provided with a self-actuating seal as described above and the upper and lower holding plates is chosen that the chamber is held tight between the plates when pressurized but can be manually inserted freely before commencement of sterilization and removed freely after sterilization by means of a gripping handle 84 when the chamber is vented or evacuated. When the interior channel 70 of self-actuating seal 68 is pressurized, the extending arms of the seal bulge slightly away from each other to exert oppositely directed forces on lid 10'a and tray 10'b, forcing them against insulating plates 82a and 82b.

As illustrated in Figures 17 and 18, holder 80 may be assembled as a modular unit in which the chamber can be slid between the holding plates and plugged into a socket in an integral rear wall 81c of the unit, holding the inlet conduit 18 and outlet conduit 16 connected to the rest of the sterilization apparatus, such as that illustrated in Figure 1. It has been found with a system constructed with the sterilization system of the invention and the preferred embodiment of cassette chamber that the sterilization and drying of surgical or dental instruments can be efficiently completed within a few minutes.

It will be understood that various forms of injector pumps or valves may be employed in the system of the invention. Electrically controlled valves, temperature sensors and eductor pumps, as well as equivalent devices, are readily available commercially and their manner of operation well known in the art. It is the combination of elements and their manner of control which the present teaching provides which provides the unique aspects of the invention. Accordingly, reference should be made to the appended claims in evaluating the scope thereof.

## Claims

1. Apparatus for steam sterilizing medical articles like dental or surgical instruments comprising an electrically heated boiler (20) and a pressure chamber (10) for housing the articles to be sterilized, said pressure chamber having a steam inlet port (12', 13) and an outlet port (14', 14) with a switchable valve (V), said boiler (20) and said chamber (10) being in communication with each other and comprising control means (38) for controlling the sterilization process within the pressure chamber (10), characterized in that
   - a pulsewise actuable pump means (26) is connecting a liquid container (30) with said boiler (20) by ducts (24, 28),
   - said pressure chamber (10) is pluggably housed in a rigid holder (11),
   - said steam inlet port (12', 13) of said pressure chamber (10) is pluggably sealed connected with a steam injection conduit (18) fixed in said rigid holder (11),
   - said injection conduit (18) is communicating with said boiler (20),
   - said steam outlet port (14', 14) of said pressure chamber (10) is pluggably sealed connected with a steam outlet conduit (16),
   - said valve (V) is arranged to said outlet conduit (16) and
   - temperature sensors (34b, 34a) are associated with said boiler (20) and said pressure chamber (10) being connected to said control means (38) for supplying data for initating and controlling sequentially said sterilization process by said control means (38) which is further connected to said pump means (26), said boiler (20) and said valve (V).

2. Apparatus according to claim 1, characterized in that said pump means (26) is a reciprocating plunger (26b) being electrically actuatable by a solenoid, and forced by a biasing spring (26c).

3. Apparatus according to claim 1, characterized in that said boiler (20) has an interior capacity being sufficiently small relative to the volume of liquid pulsed into it by each injection generated by said pump means (26) and a PID (proportional integral derivative) control means of the boiler is effected by said control means (38).

4. Apparatus according to claim 3, characterized in that said temperature sensor means (34b) of said boiler is a thermocouple which is arranged to project a small distance into the interior cavity of said boiler (20) and being in contact with the casing metal of said boiler fixed at a position intermediate to heating elements (20a) of said boiler (20).

5. Apparatus according to claim 3 and 4, charcterized in that the tip of said thermocouple is also in contact with vaporized liquid remaining in said boiling chamber.

6. Apparatus according to claim 3 to 5, characterized in that a plurality of baffles (20b) are arranged in the interior capacity of said boiler (20) preventing splashover of a boiling liquid into said steam injection conduit (18).

7. Apparatus according to claim 1 to 5, characterized in that said liquid being vaporized in said boiler is destilated or deminarlized water.

8. Apparatus according to claim 1, characterized that said rigid holder (11) comprises a spaced pair of upper (11a) and lower (11b) rigid plates having thermally insulating opposed interior lateral surfaces presenting therebetween a space to closely receive and hold said pressure chamber (10) firmly in place when pressurized by steam and further comprising means for pluggable sealed connection of said steam inlet conduit (18) to communicate with said inlet port (12, 13) of said chamber (10) and said outlet conduit (16) to communicate with said outlet port (14', 14) of said pressure chamber (10) when said pressure chamber (10) is slid between said insulating surfaces to a limiting operating position.

9. Apparatus according to claim 1, characterized in that said steam injection conduit (18) comprises an air conduit (36) having a regulator (36a) and an actuatable valve (D) being connected with a compressed air line via conduit (22).

10. Apparatus according to claim 1, characterized in that said pressure chamber comprises a bottom tray (10b) having a perforated rack (12) for receiving said articles to be sterilized, a lid (10a) for placement over said bottom tray (10b) and perimetrical compressible sealing means (68) disposed between said tray (10b) and said lid (10a).

11. Apparatus according to claim 10, characterized in that said pressure chamber (10) is rectangular in shape and said ports (12, 13, 14', 14) are arranged near the lowermost point of said bottom tray (10b), said holder (11) being in fixed spatial orientation such that said chamber (10), when inserted therein in use, is tilted to position of the corner of said outlet port (14, 14') vertically below the three other corners of said chamber (10) allowing condensate formed during sterilization to passage from the chamber (10).

12. Apparatus according to claim 10 and 11, characterized in that said perimetrical compressible sealing means (68) of the pressure chamber (10) is configured to define a channel (10'c) which is closed between the wall of said bottom tray (10'b) and the wall of said lid (10'a) in the region of a first end of the chamber (10') and which partly opens into the interior of the pressure chamber (10') at a second end of the chamber remote from said first end whereby said steam inlet port (12') is located at said first end of the chamber (10') and communicates with the interior of said channel and said outlet port (14') is located near said steam inlet port (12') and communicates with the interior of said chamber near the bottommost point thereof, such that steam introduced into said inlet port (12') at said first end of the chamber (10') proceeding along the closed portion of the channel and is distributed into the chamber (10') through the open portion of the channel remote from said inlet port (12'), passing about the articles to be sterilized, and is venting through said outlet port (14').

13. Apparatus according to claim 10 to 12, characterized in that the lower portion of said lid (10'a) of said pressure chamber (10') is stepped outwardly to form a perimetrical recess, and said sealing means (68) is an elongate resilient member of generally J-shaped cross-section seated within said perimetrical recess of the lid and has upper (68a) and lower (68b) transverse sealing edges projecting slightly outwardly from said perimetrical recess, both of said sealing edges being in sealing contact with the outer wall surface of said bottom tray (10'b) in the region of said first end of the chamber (10'), forming said closed channel, and only the lower sealing edge (68b) being in sealing contact with the outer wall surface of said bottom tray (10'b) in the region of said second end of the chamber, to form said partly open channel.

14. Apparatus according to claim 10 to 13, characterized in that said perimetrical recess of said pressure chamber (10') is tilted downwardly with respect to the upper edge of said bottom tray (10'b) from said second end of the chamber (10') to the first end thereof, whereby said sealing member (68) lies entirely below the top edge of said bottom tray (10'b) in the region of said first end of the pressure chamber (10') and partly above the top edge of the bottom tray (10'b) in the region of said second end of the pressure chamber (10').

15. Apparatus according to claim 10 to 14 characterized in that said temperature sensor (34a) is a thermocouple and inserted in said outlet conduit (16, 16') so that said temperature sensor (34a) extend a short distance into the interior of said pressure chamber (10') through a peripherally sealed aperture of a channel member (78) being tilted downwardly with respect to the upper edge of said bottom tray (10b) about the transverse axis (MM) and hold by the plane of said lid channel (10'c).

16. Apparatus according to claim 1 to 15, characterized in that said control means (38) for controlling the apparatus has a microcontroller (38)

    - being synchronized by a timer clock (44)

- having a program memory (40) and a working memory (42)
- being connected to a display (63) and a keyboard (61)
- having interfaces (56, 50) for said temperature sensor (34b) of said boiler (20) and said temperture sensor (34a) of said pressure chamber (48) and
- having interfaces (58, 60, 62) being connected to said pump means (26), the heating elements (20a) of said boiler and said valve (V).

17. Method for steam sterilization medical articles like dental or surgical instruments by using apparatus according claims 1 to 16, characterized by the following steps

1) switching said valve (V) in open position;

2) heating up said boiler (20) to around 100°C monitored by the boiler temperature (TBLR) being measured by said temperature sensor (34b);

3) pulsewise injecting controlled quantity of liquid into the hot boiler (20) by actuating said pump means (26) whenever dryness of the boiler occurs which is indicated by an abrupt increasing of the boiler temperature (TBLR) being measured by said temperature sensor (34b);

4) closing said valve (V) when said pressure chamber temperature (TCHM) has risen up to around 100°C being monitored by said temperature sensor (34a) of said pressure chamber (20);

5) heating up said boiler (20) to the sterilization temperature and pulsewise injecting a controlled quantity of liquid into the hot boiler by actuating said pump means (26), whenever dryness of said boiler (20) occurs being indicated by a sharp increase in the slop of the boiler temperature (TBLR) being measured by said temperature sensor (34b);

6) continuing the pulsed injection of discret doses of liquid by said pump (26) controlled by using the temperatur difference of boiler temperature (TBLR) and pressure chamber temperature (TCHM) meassured by said temperature sensor (34b, 34a) until the sterilization temperature is reached in said pressure chamber (10);

7) opening said valve (V) for a fixed interval for purging condensate in said pressure chamber (20) unless a drop in the chamber temperature (TCHM) is sensed by said temperature sensor (34a) of said pressure chamber;

8) monitoring the sterilization temperature of said pressure chamber (20) meassured by said temperature sensor (34a) and continuing at step 6) until the pressure chamber temperature (TCHB) is up to the sterilization temperature;

9) controlling the sterilization pressure over the sterilization process by maintaining the sterilization temperature substantially constant by pulsewise delivery of controlled quantities of liquid into said boiler (20);

10) purging condensate by opening said valve (V) for a fixed interval and continuing at step 9) until sterilization time is over;

11) turning off said heating means (20a) of said boiler (20) and opening said valve (V) for returing to atmospheric pressure of said pressure chamber.

18. Method for steam sterilization medical articles like dental or surgical instruments according to claim 17, characterized by inserting the following steps after step 7 of claim 17

- involving a loop wherein the value of the difference of said boiler temperature (TBLR) and said pressure chamber temperature (TCHM) by taking a running average of both temperatures is compared to a predetermined limit whereby said value is indicating the presence of residual air in said pressure chamber
- opening said valve (V) for a short fixed period purging said residual air
- start at step 5 of claim 17 while said pressure chamber temperature (TCHB) do not depart markedly from said boiler temperature (TBLR)

19. Method for steam sterilization medical articles like dental or surgical instruments according to claim 15 or 16, characterized by the additional following step after step 11 of claim 17

- opening said valve (D) of said compressed air line hastening, cooling and drying the instruments to be sterilized while filtered clean air is passing over.

20. Apparatus according to claim 1 to 19 for producing a vapor from a liquid comprising boiler means for vaporizing said liquid, pump means for supplying discret doses of said liquid to said boiler means, control means for detecting when substantially all of said liquid supplied to said boiler means has been vaporized and for causing said pump means to supply a said dose of said liquid to said boiler means, whereby heating power available to said boiler is maximally employed in the production of said vapor.

21. Apparatus according to claim 1 to 20 wherein said means for determining the temperature of said boiler comprises a thermocouple in contact with said chamber of said boiler.

**Patentansprüche**

1. Anordnung zur Dampfsterilisation von medizinischen Geräten, wie zahnmedizinische oder chirurgische Instrumente, mit einem Elektroboiler (20) und einer Druckkammer (10) zur Aufnahme des Sterilisationsgutes, wobei die Druckkammer (10) eine Dampfeintrittsöffnung (12',13) und eine Dampfaustrittsöffnung (14',14) mit einem Schaltventil (V) aufweist und Boiler (20) und Druckkammer (10) miteinander kommunizieren und mit einer Steuervorrichtung (38) zur Steuerung des Sterilisationsvorganges in der Druckkammer (10) vorgesehen ist, **gekennzeichnet durch**
   - eine pulsgesteuerte Pumpe (26), die einen Flüssigkeitsbehälter (30) mit dem Boiler (20) über Leitungen (24, 28) verbindet,
   - eine starre Aufnahme (11) für die als Einschubteil ausgebildete Druckkammer (10),
   - eine abdichtende steckbare Verbindung zwischen der die Dampfeintrittsöffnung (12',13) der Druckkammer (10) und einer in der starren Aufnahme (11) befestigten Eintrittsleitung (18) für Druckdampf,
   - wobei die Eintrittsleitung (18) mit dem Boiler (20) verbunden ist,
   - eine abdichtend steckbare Verbindung zwischen der Dampfaustrittsöffnung (14',14) der Druckkammer (10) und einer Dampfaustrittsleitung (16),
   - wobei das Schaltventil (V) der Austrittsleitung (16) zugeordnet ist,
   - dem Boiler (20) zugeordnete Temperaturaufnehmer (34b,34a) und durch
   - eine datenliefernde Verbindung zwischen Druckkammer (10) und Steuervorrichtung (38) zwecks sequentieller Ansteuerung des Sterilisationsvorganges durch die Steuervorrichtung (38), die zudem mit der Pumpe (26), dem Boiler (20) und dem Ventil (V) verbunden ist.

2. Anordnung nach Anspruch 1, **dadurch gekennzeichnet**, daß die Pumpe (26) einen über einen Tauchmagneten betätigbaren ein- und ausfahrbaren und von einer Feder (26c) beaufschlagten Kolben (26b) aufweist.

3. Anordnung nach Anspruch 1, **dadurch gekennzeichnet**, daß das Fassungsvermögen des Boilers (20) bezüglich der jeweils über die Pumpe (26) eingespritzten Flüssigkeitsmenge ausreichend klein gehalten ist und daß ein PID-Glied (proportional integral derivative) über die Steuervorrichtung (38) ansteuerbar ist.

4. Anordnung nach Anspruch 3, **dadurch gekennzeichnet**, daß der Temperaturaufnehmer (34b) des Boilers ein geringfügig in den Boilerinnenraum hineinragendes Thermoelement ist, das mit dem Metallmantel des Boilers an einem zwischen den Heizelementen (20a) des Boilers liegenden Punkt in Berührung steht.

5. Anordnung nach den Ansprüchen 3 und 4, **dadurch gekennzeichnet**, daß die Spitze des Thermoelementes auch mit dem im Boiler verbliebenen gesättigten Wasserdampf in Verbindung steht.

6. Anordnung nach den Ansprüchen 3 bis 5, **gekennzeichnet** durch mehrere Prallplatten (20b) im Innenraum des Boilers (20), die den Übertritt kochender Flüssigkeit in die Dampfeinspritzleitung (18) verhindern.

7. Anordnung nach den Ansprüche 1 bis 5, **dadurch gekennzeichnet**, daß die im Boiler zu verdampfende Flüssigkeit destilliertes oder entmineralisiertes Wasser ist.

8. Anordnung nach Anspruch 1, **dadurch gekennzeichnet**, daß die starre Aufnahme (11) aus einem starren Plattenpaar mit im Abstand zueinander angeordneter oberer (11a) und unterer Platte (11b) besteht, deren einander zugewandte seitliche Innenflächen thermisch isoliert sind und die zwischen sich einen Raum bilden, in den die Druckkammer (10) bei Druckbeaufschlagung durch Dampf fest gehalten ist und ferner Mittel aufweist, die ein abdichtendes steckbares Einsetzen der mit der Eintrittsöffnung (12,13) der Kammer (10) kommunizierenden Dampfeinlaßleitung (18) und der mit der Austrittsöffnung (14',14) der Druckkammer (10) kommunizierenden Austrittsleitung (16) ermöglichen, wenn die Druckkammer (10) zwischen den Isolierflächen bis zum Anschlag in ihre Betriebsstellung eingeschoben ist.

9. Anordnung nach Anspruch 1, **dadurch gekennzeichnet**, daß die Dampfeinspritzleitung (18) eine Luftleitung (36) mit einem Regulator (36a) und einem betätigbaren Ventil (D) aufweist, das über eine Leitung (22) an eine Druckluftleitung angeschlossen ist.

10. Anordnung nach Anspruch 1, **dadurch gekennzeichnet**, daß die Druckkammer eine Bodenwanne (10b) mit einem perforierten Gestell (12) zur Aufnahme des Sterilisationsgutes sowie einen die Bodenwanne abdeckenden Deckel (10a) und eine zwischen Bodenwanne (10b) und Deckel (10a) umlaufende Dichtung (68) umfaßt.

11. Anordnung nach Anspruch 10, **dadurch gekennzeichnet**, daß die Druckkammer (10) rechteckförmig ausgebildet ist und daß die Öffnungen (12,13,14',14) im untersten Punkt der Bodenwanne (10b) angeordnet sind, daß die Aufnahme (11) in einer derart festen Lage angeordnet ist, daß die Druckkammer (10) nach dem Einschieben eine Schräglage einnimmt, in der die Austrittsöffnung (14,14') senkrecht unterhalb der drei anderen Ecken der Kammer (10) liegt und den Abfluß von sich im Zuge der Sterilisation bildendes Kondensat aus der Kammer (10) ermöglicht.

12. Anordnung nach den Ansprüchen 10 und 11, **dadurch gekennzeichnet**, daß die umlaufende, zusammendrückbare Dichtung (68) der Druckkammer (10) so ausgebildet ist, daß ein zwischen der Wandung der Bodenwanne (10'b) und der Wand des Deckels (10'a) geschlossener Kanal (10'c) im Bereich eines ersten Endes der Kammer (10') entsteht, der sich in Teilen zum Innern der Druckkammer (10') an einem zweiten vom ersten Ende abliegenden Ende öffnet, so daß die Dampfeintrittöffnung (12') am ersten Ende der Kammer (10') liegt und mit dem Innenraum des Kanals kommuniziert und daß die Austrittsöffnung (14') im Bereich der Dampfeinlaßöffnung (12') liegt und mit dem Kammerinnenraum nahe ihrem tiefsten Punkt in Verbindung steht, so daß in die Eintrittsöffnung (12') am ersten Ende der Kammer (10') gelangender Dampf im geschlossenen Teil des Kanals entlangströmt und durch den offenen Teil des Kanals an einer von der Eintrittsöffnung (12') im Abstand liegenden Stelle in die Kammer (10') gelangt, dort das Sterilisationsgut überstreicht und durch die Austrittsöffnung (14') entweicht.

13. Anordnung nach den Ansprüchen 10 bis 12, **dadurch gekennzeichnet**, daß der untere Teil des Deckels (10'a) der Druckkammer (10') stufenförmig nach außen vorsteht und eine umlaufende Rinne bildet, daß die Dichtung (68) ein langgestrecktes elastisches Teil von im wesentlichen J-förmigem Querschnitt ist, das in die umlaufende Rinne des Deckels eingelegt ist und obere (68a) und untere (68b) stirnseitige Dichtkanten aufweist, die etwas über die umlaufende Rinne vorstehen, wobei beide Dichtkanten an der Außenwand der Bodenwanne (10'b) im Bereich des ersten Endes der Kammer (10') dichtend anliegen und den geschlossenen Kanal bilden, wobei nur die untere Dichtkante (68b) auch an der Außenwandung der Bodenwanne (10'b) im Bereich des zweiten Endes der Kammer dichtend anliegt und somit den teilweise offenen Kanal bildet.

14. Anordnung nach den Ansprüchen 10 bis 13, **dadurch gekennzeichnet**, daß die umlaufende Aufnahme der Druckkammer (10') in bezug auf die Oberkante der Bodenwanne (10'b) von zweiten Ende der Kammer (10') zu ihrem ersten Ende hin nach unten zu schräg verläuft, derart, daß die Dichtung (68) im Bereich des ersten Endes der Druckkammer (10') gänzlich unterhalb der Oberkante der Bodenwanne (10'b) und im Bereich des zweiten Endes der Druckkammer (10') teilweise oberhalb der Oberkante der Bodenwanne (10'b) liegt.

15. Anordnung nach den Ansprüchen 10 bis 14, **dadurch gekennzeichnet**, daß der Temperaturaufnehmer (34a) ein in die Austrittsleitung (16,16') eingesetztes Thermoelement ist, das mit einem kleinen Teil in den Innenraum der Druckkammer (10') hineinragt und eine abgedichtete Öffnung in einem Kanal (78) durchsetzt, der in bezug auf die Oberkante der Bodenwanne (10b) um die Querachse (MM) geneigt und von der Ebene des Deckelkanals (10'c) gehalten ist.

16. Anordnung nach den Ansprüchen 1 bis 15, **dadurch gekennzeichnet**, daß die Steuermittel (38) für die Steuerung der Vorrichtung einen Mikrocontroller (38) aufweist, der
- von einem Taktgeber (44) synchronisiert ist,
- einen Programmspeicher (40) und einen Arbeitsspeicher (42) aufweist,
- mit einem Display (63) und einer Tastatur (61) verbunden ist,
- Schnittstellen (56,50) für den Temperaturaufnehmer (34b) des Boilers (20) und für den Temperaturaufnehmer (34a) der Druckkammer (48) aufweist sowie ferner
- Schnittstellen (58, 60, 62) aufweist, die mit der Pumpe (26), den Heizelementen (20a) des Boilers und mit dem Ventil (V) verbunden sind.

**17.** Verfahren zur Dampfsterilisation von medizinischen Geräten, wie zahnmedizinische oder chirurgische Instrumente unter Verwendung der Anordnung nach den Ansprüchen 1 bis 16, **gekennzeichnet durch** folgende Verfahrensschritte:

1) Einstellen des Schaltventils (V) auf Stellung AUF;

2) Aufheizen des Boilers (20) auf etwa 100°C unter Überwachung durch das Glied (TBLR) entsprechend den Meßwerten des Temperaturaufnehmers (34b);

3) pulsweises Einspritzen einer eingestellten Flüssigkeitsmenge in den heißen Boiler (20) durch Betätigen der Pumpe (26) sobald durch abrupten Anstieg der Boilertemperatur (TBLR) entsprechend den Meßwerten des Temperaturaufnehmers (34b) Trockenheit im Boiler angezeigt ist;

4) Schließen des Schaltventils (V), wenn die Temperatur in der Druckkammer (TCHM) etwa 100°C erreicht hat, was durch den Temperaturaufnehmer (34a) der Druckkammer (20) überwacht wird;

5) Aufheizen des Boilers (20) auf die Sterilisationstemperatur und pulsweises Einspritzen einer eingestellten Flüssigkeitsmenge in den heißen Boiler durch Betätigung der Pumpe (26) sobald Feuchtigkeitsmangel im Boiler (20) auftritt, was durch einen steilen Anstieg in der Boilertemperaturkurve (TBLR) nach den Meßwerten des Temperaturaufnehmers (34b) angezeigt wird,

6) Weiterhin taktweises Einspritzen dosierter Flüssigkeit durch die Pumpe (26) gesteuert unter Verwendung der Temperaturdifferenz zwischen Boilertemperatur (TBLR) und Druckkammertemperatur (TCHM) über die Meßwerte des Temperaturaufnehmer (34b, 34a) bis zum Erreichen der Sterilisationstemperatur in der Druckkammer (10),

7) Öffnen des Schaltventils (V) über einen bestimmten Zeitraum zwecks Ablassens von Kondensat in der Druckkammer (20), sofern kein Temperaturabfall in der Kammer (TCHM) durch den Temperaturaufnehmer (34a) in der Druckkammer angezeigt wird,

8) Überwachen der Sterilisationstemperatur in der Druckkammer (20) entsprechend den Meßwerten des Temperaturaufnehmers (34a) und Fortsetzung mit Verfahrensschritt 6) bis zum Erreichen der Sterilisationstemperatur (TCHB) in der Druckkammer,

9) Steuerung des Sterilisationsdruckes während des Sterilisationsvorganges durch Aufrechterhalten einer im wesentlichen konstanten Sterilisationstemperatur durch pulsweises Zuführen eingestellter Flüssigkeitsmengen in den Boiler (20),

10) Ablassen von Kondensat durch öffnen des Schaltventils (V) über einen bestimmten Zeitraum und Fortsetzen des Vorgangs mit Verfahrensschritt 9) bis zum Ende der Sterilisationszeit,

11) Abschalten der Heizvorrichtung (20a) für den Boiler (20) und Öffnen des Schaltventils (V) um die Druckkammer auf Atmosphärendruck zurückzuführen.

**18.** Verfahren zur Dampfsterilisation von medizinischen Geräten, wie zahnmedizinische oder chirurgische Instrumente nach Anspruch 17, **gekennzeichnet durch** folgende Verfahrensschritte nach Verfahrensschritt 7 gemäß Anspruch 17

- Einführen einer Regelschleife, in der der Wert der Differenz von Boilertemperatur (TBLR) und Druckkammertemperatur (TCHM) über einen laufend ermittelten Mittelwert beider Temperaturen mit einem vorbestimmten Grenzwert verglichen wird, so daß das Vorhandensein von Restluft in der Druckkammer angezeigt wird,

- Öffnen des Ventils (V) für eine kurze eingestellte Zeit zwecks Ablassens der Restluft,

- Weiter nach Verfahrensschritt 5 gemäß Anspruch 17 solange die Druckkammertemperatur (TCHB) nicht signifikant von der Boilertemperatur (TBLR) abweicht.

**19.** Verfahren zur Dampfsterilisation von medizinischen Geräten, wie zahnmedizinische oder chirurgische Instrumente nach den Ansrpüchen 15 oder 16 **gekennzeichnet durch** folgenden nach Verfahrensschritt 11 gemäß Anspruch 17 aufzunehmenden weiteren Verfahrensschritt:

- Öffnen des Ventils (D) der Druckluftleitung zwecks Zufuhr von gefilterter Frischluft zum schnellen Abkühlen und Trocknen der sterilisierten Instrumente.

**20.** Anordnung nach den den Ansprüchen 1 bis 19 zur Erzeugung von Flüssigkeitsdampf mit einem Boiler zum Verdampfen der Flüssigkeit mit einer Pumpe zur Zufuhr von getrennten, dosierten Flüssigkeitsmengen in den Boiler sowie mit Mitteln zur Detektion der jeweils noch für die Verdampfung zur Verfügung stehenden Flüssigkeitsmenge im Boiler und zur Ansteuerung der Pumpe zwecks Zufuhr einer dosierten Flüssigkeitsmenge in den Boiler, alles unter maximaler Reduzierung des Energieverbrauchs für die Dampferzeugung im Boiler.

**21.** Anordnung nach den Ansprüchen 1 bis 20, **dadurch gekennzeichnet**, daß das Mittel zur Messung der Temperatur im Boiler aus einem Thermoelement besteht, das mit dem Boilerinnenraum in Berührung steht.

## Revendications

**1.** Appareil pour stériliser à la vapeur des articles médicaux tels que des instruments dentaires ou chirurgicaux, comprenant un chauffe-eau électrique (20) et une chambre à pression (10) pour contenir les articles devant être stérilisés, cette chambre à pression ayant un orifice d'admission de vapeur (12',13) et un orifice de sortie (14',14) avec une vanne commutable (V), le chauffe-eau (20) et la chambre (10) étant en communication l'un avec l'autre et comprenant un moyen de commande (38) pour commander le processus de stérilisation dans la chambre à pression (10), caractérisée en ce qu'un moyen de pompage (26) actionnable d'une manière pulsatoire relie un conteneur de liquide (30) au chauffe-eau (20), par l'intermédiaire de conduits (24,28), la chambre à pression (10) est logée d'une manière enfichable dans un support rigide (11), l'orifice d'admission de vapeur (12',13) de las chambre à pression (10) est connecté, d'une manière étanche et enfichable, à un conduit d'injection de vapeur (18) fixé dans le support rigide (11), ce conduit d'injection (18) communique avec le chauffe-eau (20), l'orifice de sortie de vapeur (14',14) de la chambre à pression (10) est connecté, d'une manière étanche et enfichable, à un conduit de sortie de vapeur (16), la vanne (V) est montée sur le conduit de sortie de vapeur (16) et des capteurs de température (34b,34a) sont associés au chauffe-eau (20) et la chambre à pression (10) et ils sont connectés au moyen de commande (38) pour fournir des données pour le démarrage et la commande séquentielle du processus de stérilisation par le moyen de commande (38) qui est en outre connecté au moyen de pompage (26), au chauffe-eau (20) et à la vanne (V).

**2.** Appareil suivant la revendication 1 caractérisé en ce que le moyen de pompage (26) est un plongeur (26b) à mouvement alternatif actionné électriquement par un solénoïde et sollicité par un ressort de rappel (26c).

**3.** Appareil suivant la revendication 1 caractérisé en ce que le chauffe-eau (20) a une capacité interne suffisamment petite par rapport au volume de liquide introduit par chaque injection produite par le moyen de pompage (26) et un moyen de commande du chauffe-eau, du type proportionnel, intégral et dérivé, est effectué par le moyen de commande (38).

**4.** Appareil suivant la revendication 3 caractérisé en ce que le capteur de température (34b) du chauffe-eau est un thermocouple qui est disposé de manière à faire saillie sur une courte distance dans la cavité interne du chauffe-eau (20) et qui est en contact avec l'enveloppe métallique du chauffe-eau en étant fixé dans une position intermédiaire entre des éléments de chauffage (20a) du chauffe-eau (20).

**5.** Appareil suivant l'une quelconque des revendications 3 et 4 caractérisé en ce que l'extrémité du thermocouple est également en contact avec le liquide vaporisé demeurant dans la chambre d'ébullition.

**6.** Appareil suivant l'une quelconque des revendications 3 à 5 caractérisé en ce qu'une pluralité de chicanes (20b) sont disposées dans la capacité interne du chauffe-eau (20), afin d'empêcher une projection d'un liquide en ébullition vers et dans le conduit d'injection de vapeur (18).

**7.** Appareil suivant l'une quelconque des revendications 1 à 5 caractérisé en ce que le liquide qui est vaporisé dans le chauffe-eau est de l'eau distillée ou déminéralisée.

**8.** Appareil suivant la revendication 1 caractérisé en ce que le support rigide (11) comprend une paire de plaques rigides supérieure (11a) et inférieure (11b), espacées l'une de l'autre, ayant des surfaces latérales internes opposées, isolantes thermiquement, délimitant entre elles un espace de manière à recevoir étroitement et à maintenir fermement en place la chambre à pression (10) lorsqu'elle est mise sous pression par de la vapeur, et il comprend en outre un moyen pour établir une connexion, d'une manière étanche et enfichable, du conduit d'admission de vapeur (18), pour qu'il communique avec l'orifice d'admission (12,13) de la chambre (10), et du conduit de sortie (16) pour qu'il communique avec l'orifice de sortie (14',14) de la chambre à pression (10), lorsque cette chambre à pression (10) est glissée entre les surfaces isolantes jusqu'à une position opérationnelle limite.

**9.** Appareil suivant la revendication 1 caractérisé en ce que le conduit d'injection de vapeur (18) comprend un conduit d'air (36) ayant un régulateur (36a) et une vanne actionnable (D) reliée à une conduite d'air comprimé par l'intermédiaire d'un conduit (22).

10. Appareil suivant la revendication 1 caractérisé en ce que la chambre à pression comprend un bac inférieur (10b) présentant une grille (12) pour recevoir les articles devant être stérilisés, un couvercle (10a) destiné à être placé pardessus le bac inférieur (10b) et un moyen d'étanchéité périphérique compressible (68) disposé entre le bar (10b) et le couvercle (10a).

11. Appareil suivant la revendication 10 caractérisé en ce que la chambre à pression (10) a une forme rectangulaire et les orifices (12,13,14',14) sont situés à proximité du point le plus bas du bac inférieur (10b), le support (11) étant dans une orientation spatiale fixe telle que la chambre (10), lorsqu'elle est introduite dans ce support en cours d'utilisation, soit basculée vers la position du coin de l'orifice de sortie (14,14') se trouvant verticalement en dessous des trois autres coins de la chambre (10), afin de permettre au condensat formé pendant la stérilisation de s'écouler à partir de la chambre (10).

12. Appareil suivant l'une quelconque des revendications 10 et 11 caractérisé en ce que le moyen d'étanchéité périphérique (68), compressible, de la chambre à pression (10) est configuré de manière à définir un canal (10'c) qui est fermé entre la paroi du bac inférieur (10'b) et la paroi du couvercle (10'a), dans la région d'une première extrémité de la chambre (10'), et qui est partiellement ouvert vers et dans l'intérieur de la chambre à pression (10'), à l'endroit d'une seconde extrémité de la chambre éloignée de sa première extrémité, de telle façon que l'orifice d'admission de vapeur (12') soit situé à la première extrémité de la chambre (10') et qu'il communique avec l'intérieur du canal, et que l'orifice de sortie (14') soit situé à proximité de l'orifice d'admission de vapeur (12') et qu'il communique avec l'intérieur de la chambre à proximité du point le plus bas de celle-ci, si bien que de la vapeur introduite dans l'orifice d'admission (12'), à la première extrémité de la chambre (10'), s'écoule le long de la portion fermée du canal et est distribuée dans la chambre (10') à travers la portion ouverte de ce canal qui est éloignée de l'orifice d'admission (12'), en passant autour des articles devant être stérilisés, et que cette vapeur est évacuée à travers l'orifice de sortie (14').

13. Appareil suivant l'une quelconque des revendications 10 à 12 caractérisé en ce que la partie inférieure du couvercle (10'a) de la chambre à pression (10') est étagée vers l'extérieur de manière à former un creux périphérique et le moyen d'étanchéité (68) est un organe élastique allongé, ayant une section transversale généralement en forme de J, logée dans le creux périphérique du couvercle et il présente des bords d'étanchement transversaux supérieur (68a) et inférieur (68b), faisant légèrement saillie vers l'extérieur à partir du creux périphérique, ces deux bords d'étanchement étant en contact étanche avec la surface externe de la paroi du bac inférieur (10'b) dans la région de la première extrémité de la chambre (10'), en formant le canal fermé, et seul le bord d'étanchement inférieur (68b) étant en contact étanche avec la surface externe de la paroi du bac inférieur (10'b) dans la région de la seconde extrémité la chambre, afin de former le canal partiellement ouvert.

14. Appareil suivant l'une quelconque des revendication 10 à 13 caractérisé en ce que le creux périphérique de la chambre à pression (10') est incliné vers le bas par rapport au bord supérieur du bac inférieur (10'b), à partir de la seconde extrémité de la chambre (10') et en direction de sa première extrémité, de telle façon que l'organe d'étanchéité (68) soit situé entièrement en dessous du bord supérieur du bac inférieur (10'b) dans la région de la première extrémité de la chambre à pression (10') et qu'il soit situé partiellement au-dessus du bord supérieur du bac inférieur (10'b) dans la région de la seconde extrémité de la chambre à pression (10').

15. Appareil suivant l'une quelconque des revendications 10 à 14 caractérisé en ce que le capteur de température (34a) est un thermocouple et il est inséré dans le conduit de sortie (16,16') de telle façon que ce capteur de température (34a) s'étende sur une courte distance vers l'intérieur de la chambre à pression (10'), à travers une ouverture, fermée d'une manière étanche à la périphérie, d'un organe profilé (78) qui est incliné vers le bas par rapport au bord supérieur du bac inférieur (10b) autour de l'axe transversal (MM), et qui est maintenu par le plan du profilé (10'c) du couvercle.

16. Appareil suivant l'une quelconque des revendications 1 à 15 caractérisé en ce que le moyen de commande (38) pour la commande de l'appareil comporte un microcontrôleur (38) qui est synchronisé par une horloge (44), qui a une mémoire de programme (40) et une mémoire de travail (42), qui est connectée à un afficheur (63) et à un clavier (61), qui a des interfaces (56,50) pour le capteur de température (34b) du chauffe-eau (20) et le capteur de température (34a) de la chambre à pression (48) et qui a des interfaces (58,60,62) connectées au moyen de pompage (26), aux éléments de chauffage (20a) du chauffe-eau et à la vanne (V).

17. Procédé de stérilisation à la vapeur d'articles tels que des instruments dentaires ou chirurgicaux, en utilisant un appareil suivant les revendications 1 à 16, caractérisé en ce qu'il comprend les étapes suivantes consistant :

1) à commuter la vanne (V) dans sa position d'ouverture;

2) à chauffer le chauffe-eau (20) à une température d'environ 100°, ce chauffe-eau étant piloté par la mesure de la température (TBLR) du chauffe-eau mesurée par le capteur de température (34b);

3) à injecter d'une manière pulsatoire une quantité déterminée de liquide dans le chauffe-eau (20), par suite de l'actionnement du moyen de pompage (26), chaque fois que le chauffe-eau se trouve être vidé ou à sec, ce qui est indiqué par un accroissement brutal de la température (TBLR) du chauffe-eau mesurée par le capteur de température (34b);

4) à fermer la vanne (V) lorsque la température (TCHM) de la chambre à pression s'est élevée jusqu'à environ 100°C, en étant surveillée par le capteur de température (34a) de la chambre à pression (20);

5) à chauffer le chauffe-eau (20) jusqu'à la température de stérilisation et à injecter d'une manière pulsatoire une quantité déterminée de liquide dans le chauffe-eau, par suite de l'actionnement du moyen de pompage (26), chaque fois que le chauffe-eau vient à être vidé et sec, ce qui est indiqué par un accroissement brutal de la pente de la température (TBLR) du chauffe-eau telle que mesurée par le capteur de température (34b);

6) à continuer l'injection pulsée de doses discrètes de liquide par le moyen de pompage (26) commandé en utilisant la différence entre la température (TBLR) du chauffe-eau et la température (TCHM) de la chambre à pression mesurées par les capteurs de température (34b,34a), jusqu'à ce que la température de stérilisation soit atteinte dans la chambre à pression (10);

7) à ouvrir la vanne (V) pendant un intervalle de temps fixe afin de purger le condensat dans la chambre à pression (20) à moins qu'une chute de la température (TCHM) de la chambre ne soit détectée par le capteur de température (34a) de la chambre à pression;

8) à surveiller la température de stérilisation de la chambre à pression (20), mesurée par le capteur de température (34a), et à continuer à l'étape 6) jusqu'à ce que la température (TCHM) de la chambre à pression ait atteint la température de stérilisation;

9) à commander la pression de stérilisation, pendant le processus de stérilisation, en maintenant la température de stérilisation sensiblement constante, par une fourniture pulsée de quantités commandées de liquide vers et dans chauffe-eau (20);

10) à purger le condensat en ouvrant la vanne (V) pendant un intervalle de temps fixe et à continuer à l'étape (9) jusqu'à ce que la période de stérilisation soit achevée;

11) à couper les moyens des chauffage (20a) du chauffe-eau (20) et à ouvrir la vanne (V) pendant le retour de la chambre à pression à la pression atmosphérique.

18. Procédé de stérilisation à la vapeur d'articles médicaux tels que des instruments dentaires ou chirurgicaux suivant la revendication 16 caractérisé par l'insertion, après l'étape (7) de la revendication 17, des étapes suivantes consistant à réaliser une boucle dans laquelle la valeur de la différence entre la température (TBLR) du chauffe-eau et la température (TCHM) de la chambre à pression en prenant une moyenne en cours de ces deux températures, est comparée à une limite prédéterminée de telle façon que cette valeur indique la présence d'air résiduel dans la chambre à pression, à ouvrir la vanne (V) pendant une courte période de temps constante pour purger l'air résiduel et à démarrer à l'étape (5) de la revendication 17 tant que la température (TCHM) de la chambre à pression ne s'écarte pas d'une manière notable de la température (TBLR) du chauffe-eau.

19. Procédé de stérilisation à la vapeur d'articles médicaux tels que des instruments dentaires ou chirurgicaux suivant la revendication 15 ou 16 caractérisé par l'étape additionnelle suivante, après l'étape (11) de la revendication 17, consistant à ouvrir la vanne (D) de la canalisation d'air comprimé pour accélérer, refroidir et sécher les instruments devant être stérilisés tandis que de l'air pur filtré passe sur eux.

**20.** Appareil suivant l'une quelconque des revendications 1 à 19 pour produire une vapeur à partir d'un liquide, comprenant un chauffe-eau pour vaporiser le liquide, un moyen de pompage pour fournir des doses discrètes du liquide au chauffe-eau, un moyen de commande pour détecter le moment où pratiquement la totalité du liquide fourni au chauffe-eau a été vaporisée et pour amener le moyen de pompage à fournir une dose de liquide au chauffe-eau de telle façon que la puissance de chauffage disponible pour le chauffe-eau soit employée au maximum à la production de la vapeur.

**21.** Appareil suivant l'une quelconque des revendications 1 à 20 caractérisé en ce que le moyen pour déterminer la température du chauffe-eau est constitué par un thermocouple en contact avec la paroi de la cavité d'ébullition du chauffe-eau.

FIG.1.

EP 0 429 960 B1

FIG.2.

FIG.3.

FIG.4.

EP 0 429 960 B1

FIG.5.

FIG.6.

FIG. 7

FIG. 8

FIG. 9

FIG. 10

FIG. 11

FIG.12.

FIG.13.

EP 0 429 960 B1

FIG.14.

FIG.15.

FIG.16.

FIG.17.

FIG.18.